# EUROPÄISCHE PATENTSCHRIFT

(11) **EP 2 785 287 B1**
(45) Veröffentlichungstag und Bekanntmachung des Hinweises auf die Patenterteilung: **25.05.2016**
(21) Anmeldenummer: 12797712.2
(22) Anmeldetag: 29.11.2012
(51) Int. Cl.: A61F 2/80

(54) **EINRICHTUNG UND PROTHESENSYSTEM**
DEVICE AND PROSTHESIS SYSTEM
DISPOSITIF ET SYSTÈME DE PROTHÈSE

(30) Priorität: 29.11.2011 DE 102011119593; 14.06.2012 DE 102012011681
(43) Veröffentlichungstag der Anmeldung: 08.10.2014
(73) Patentinhaber: Otto Bock HealthCare GmbH, 37115 Duderstadt (DE)
(72) Erfinder: MOSLER, Lüder, 37115 Duderstadt (DE); HILLMANN, Martin, 37115 Duderstadt (DE); MÜLLER, André, 37115 Duderstadt (DE); SCHÖNEMEIER, Mark, 37077 Göttingen (DE)
(74) Vertreter: Lins, Edgar
(86) Internationale Anmeldenummer: PCT/EP2012/004931
(87) Internationale Veröffentlichungsnummer: WO 2013/079202

(56) Entgegenhaltungen:
- EP-A1- 1 875 881
- WO-A1-84/00881
- WO-A1-2010/036370
- DE-C- 746 781
- US-A1- 2004 260 403
- US-B1- 7 427 298

## Beschreibung

Die Erfindung betrifft eine Einrichtung zum Verbinden einer Pumpe mit einer in einem Prothesenschaft vorgesehenen Durchgangsbohrung, wobei die Einrichtung zumindest einen Kanal mit einem ersten Ausgang zum Verbinden mit der in dem Prothesenschaft vorgesehenen Auslassöffnung und einem zweiten Ausgang, der mit der Pumpe verbindbar ist, und wenigstens ein Ventil umfasst.

Eine derartige Einrichtung ist beispielsweise aus der US 5,702,489 A oder der US 2004/0260403 A1, die den nächstliegenden Stand der Technik darstellt, bekannt.

Um ein Prothesensystem sicher an einem Amputationsstumpf eines Patienten zu befestigen, wird häufig zwischen dem Amputationsstumpf bzw. einem darüber gezogenen Liner, beispielsweise aus einem Elastomer, und dem Prothesenschaft ein Unterdruck erzeugt. Zwischen dem Prothesenschaft und dem Liner, der über einen Amputationsstumpf gezogen ist, entsteht ein Innenvolumen, das es zu evakuieren gilt. Dazu verfügt der Prothesenschaft in der Regel über eine distale Durchgangsbohrung, an der eine Vakuumpumpe angeschlossen ist. Eine derartige Anordnung ist beispielsweise aus der WO 2006/135851 A2 bekannt. Dabei ist die Vakuumpumpe zusammen mit einer dafür vorgesehenen Stromversorgung Teil des Prothesenaufbaus. An dem Prothesenaufbau selbst ist ein Schalter angeordnet, durch den die Vakuumpumpe aktiviert werden kann, so dass der Träger des Prothesensystems selbst den für ihn angenehmen und für den festen Sitz des Prothesensystems erforderlichen Unterdruck einstellen kann.

Wird ein derartiges Prothesensystem über einen längeren Zeitraum, beispielsweise einen Tag, getragen, ist es möglich, dass aufgrund der Bewegung des Amputationsstumpfes und des Prothesensystems sowie durch kleine Undichtigkeiten Luft in das Innenvolumen zwischen den Liner und den Prothesenschaft eindringen kann. Dadurch lässt der Unterdruck nach, wodurch die Befestigung des Prothesensystems am Amputationsstumpf geschwächt wird.

Der Vorteil der in der WO 2006/135851 A2 vorgesehenen Ausführungsform besteht darin, dass der Träger des Prothesensystems die Vakuumpumpe immer bei sich führt, da sie Teil des Prothesensystems ist. Sollte aufgrund von eingedrungener Luft die Haftwirkung des Prothesensystems nachlassen, kann der Träger einfach über den Schalter die Vakuumpumpe aktivieren und so den optimalen Druck wiederherstellen. Wird der Druck über einen Sensor gemessen, kann die Pumpe auch automatisch aktiviert werden. Nachteilig an der gezeigten Ausführungsform ist jedoch, dass zum einen immer eine ausreichende Stromversorgung, beispielsweise durch Batterien, für die Vakuumpumpe vorgesehen sein muss und dass zum anderen sowohl das Gewicht dieser Stromversorgung als auch das der Vakuumpumpe mitgeführt werden muss. Zudem ist es möglich, dass insbesondere bei kurzen Prothesensystemen nicht genügend Raum am Prothesensystem selbst ist, um die Vakuumpumpe und ihre Stromversorgung in möglichst unauffälliger Weise unterzubringen.

Daher ist es beispielsweise aus der US 5,702,489 und der US 6,926,742 bekannt, eine externe Vakuumpumpe vorzusehen.

In der US 5,702,489 wird dafür zwischen dem Prothesenschaft und dem restlichen Prothesenaufbau ein Adapterelement angeordnet. Dieses verfügt über einen Kanal, dessen einer Ausgang mit der Durchgangsbohrung, die im Prothesenschaft vorgesehen ist, verbunden wird. Am anderen Ende des Kanals, das seitlich aus dem Adapterelement austritt, ist eine Anschlussvorrichtung vorgesehen, an der beispielsweise die Pumpe angeschlossen wird. Dazwischen ist ein Nadelventil vorgesehen, durch das der Kanal verschlossen werden kann, wenn keine Pumpe am zweiten Ausgang angeschlossen ist.

Nachteilig bei diesem System ist, dass der Träger des Prothesensystems einen eventuellen Druckverlust zwischen Liner und Prothesenschaft nur dann ausgleichen kann, wenn er eine Vakuumpumpe als separates Werkzeug mit sich führt. Zudem muss er diese Vakuumpumpe dann an die entsprechende Vorrichtung des Prothesensystems bzw. des Adapterelementes anschließen, was insbesondere für ältere oder in ihrer Mobilität eingeschränkte Träger von Prothesensystemen schwierig bis unmöglich ist. Zudem kann das Ventil, das den zweiten Ausgang des Kanals verschließt, wenn keine Pumpe angeschlossen ist, auch ohne angeschlossene Vakuumpumpe geöffnet werden. In diesem Fall dringt Luft durch den Kanal in das Innenvolumen ein, so dass die Prothese nicht mehr am Amputationsstumpf haftet.

Zumindest zur Behebung dieses letzten Nachteils wird in der WO 2010/036370 A1 vorgeschlagen, an einem Ende des Kanals ein Rückschlagventil vorzusehen, das ein Eintreten von Luft in das Innenvolumen sicher verhindert. Wird am zweiten Ausgang des Kanals eine Unterdruckpumpe angeschlossen, kann dennoch Luft aus dem Innenvolumen herausgepumpt werden, da das Rückschlagventil in dieser Richtung ein Durchströmen ermöglicht. Der Vorteil dieser Anordnung, dass keine Luft in das Innenvolumen eindringen kann, ist gleichzeitig ihr Nachteil. Insbesondere wenn der Träger des Prothesensystems die Prothese ablegen will, ist es von Vorteil, das Innenvolumen wieder mit Luft zu befüllen. Dies ist bei der dort gezeigten Ausführungsform nicht möglich, da das Rückschlagventil dies sicher verhindert. Zudem weist ein feststehendes Rückschlagventil den Nachteil auf, dass der Öffnungsdruck des Ventils die Messung des Unterdrucks verfälscht. Bei einem Rückfluss durch die Pumpe registriert die Regelung einen Abfall des Unterdrucks, den es im Schaft jedoch nicht gibt.

Der Erfindung liegt somit die Aufgabe zugrunde, eine Einrichtung sowie ein Prothesensystem bereitzustellen, das die vorgenannten Nachteile aus dem Stand der Technik behebt.

Die Erfindung löst die gestellte Aufgabe durch eine gattungsgemäße Einrichtung, bei der das Ventil in einen ersten Modus und in einen zweiten Modus bringbar ist, wobei das Ventil in dem ersten Modul ein Ein-Wege-Ventil, das nur einen Durchfluss von dem ersten Ausgang zu dem zweiten Ausgang erlaubt, und in dem zweiten Modus ein Zwei-Wege-Ventil ist, so dass Luft in beiden Richtungen durch den Kanal strömen kann, wobei jeweils das Ventil durchströmt wird. Eine derartige Einrichtung kann dabei in ein Teil eines Prothesensystems, etwa den Prothesenschaft integriert sein oder als separates Bauteil ausgebildet sein. Dieses wird dann als ein Adapterelement beispielsweise zwischen dem

Prothesenschaft und einem Prothesenaufbau angeordnet. Der erste Ausgang des wenigstens einen Kanals kann dabei entweder direkt mit einem Teil des Prothesenschaftes oder über ein Verbindungselement, beispielsweise einen Schlauch, mit der Durchgangsbohrung im Prothesenschaft verbunden werden.

Wird nun eine erfindungsgemäße Einrichtung verwendet, etwa indem sie zwischen einem Prothesenschaft und einem Prothesenaufbau angeordnet wird, kann an diese Einrichtung eine Pumpe angebracht werden. Dabei wird die Pumpe mit dem zweiten Ausgang des mindestens einen Kanals der Einrichtung verbunden. Besonders vorteilhaft ist es, wenn dadurch, dass die Pumpe angeordnet wird, das Ventil bereits in den zweiten Modus gebracht wird. Im zweiten Modus handelt es sich bei dem Ventil um ein Zwei-Wege-Ventil, so dass Luft in beide Richtungen durch den Kanal strömen kann. Wird nun ein Prothesensystem, in dem eine derartige Einrichtung zum Einsatz kommt, vom Träger angelegt, kann eine Unterdruckpumpe an den zweiten Kanalausgang der Einrichtung angeordnet werden, wodurch das Innenvolumen zwischen Prothesenschaft und Liner bzw. Amputationsstumpf evakuiert werden kann. Soll hingegen ein derartiges Prothesensystem vom Träger abgelegt werden, kann eine Überdruckpumpe an den zweiten Kanalausgang der Einrichtung angeschlossen werden. Befindet sich das Ventil in diesem Fall im zweiten Modus, handelt es sich also um ein Zwei-Wege-Ventil, kann auch Luft in das Innenvolumen zwischen Prothesenschaft und Liner bzw. Amputationsstumpf hineingepumpt werden, so dass sich der Prothesenschaft und damit das Prothesensystem leicht vom Amputationsstumpf lösen lassen. Ist jedoch keine Pumpe am zweiten Kanalausgang der Einrichtung angeordnet, befindet sich das Ventil vorteilhafterweise im ersten Modus. In diesem Fall handelt es sich bei dem Ventil um ein Ein-Wege-Ventil, das in einer besonders bevorzugten Ausgestaltung nur einen Durchfluss in Richtung von dem ersten Ausgang zu dem zweiten Ausgang des Kanals ermöglicht. In diesem Fall ist es also nur möglich, dass Luft aus dem Innenvolumen herausgedrückt wird, ohne dass Luft von außen hineingelangen kann.

Kommt es nun aufgrund von Bewegungen des Trägers des Prothesensystems oder kleinen Undichtigkeiten zu einem Eindringen von Luft in das Innenvolumen, wird diese Luft beim nächsten Belasten der Prothese aus dem Innenvolumen herausgedrückt. Dabei durchfließt sie den Kanal vom ersten Ausgang in Richtung auf den zweiten Ausgang, was auch im ersten Modus des Ventils, wenn es sich also um ein Ein-Wege-Ventil handelt, möglich ist. Damit werden alle Nachteile aus dem Stand der Technik auf besonders einfache und sichere Weise behoben. Ein im Laufe des Tages auftretender Druckverlust und eine damit nachlassende Haftwirkung werden durch diese Ausgestaltung sicher verhindert. Zudem ist durch die besondere Ausgestaltung des Ventils sowohl das Anlegen als auch das Ablegen des Prothesensystems für den Träger einfach und schnell möglich.

Als vorteilhaft hat sich herausgestellt, wenn das Ventil eine Membran umfasst. Das Ventil kann beispielsweise als Schnabel- oder Domventil und mit einfacher oder doppelter Schlitzung ausgebildet sein. Dabei ist das Ventil dann vom ersten Modus in den zweiten Modus bringbar, indem die Membran beispielsweise durch ein Durchstoßelement, das in einer besonders bevorzugten Ausführungsform einen Durchlass umfasst, durchstoßen oder durch auf die Membran aufgebrachten Druck geöffnet wird. Wird das Durchstoßelement wieder entfernt, schließt sich die Membran und das Ventil wird wieder im ersten Modus als Ein-Wege-Ventil betrieben.

Vorteilhafterweise weist der zumindest eine Kanal zudem einen dritten Ausgang zum Verbinden mit der in dem Prothesenschaft vorgesehenen Durchgangsbohrung und ein Abschlusselement auf, das mit dem dritten Ausgang verbindbar ist und in einem verbundenen Zustand ein Einströmen durch den dritten Ausgang in den wenigstens einen Kanal verhindert. Auf diese Weise ist die Einrichtung flexibel einsetzbar. So kann der erste Ausgang beispielsweise an einer dem Prothesenschaft zugewandten Seite der Einrichtung vorgesehen sein, und so direkt mit einem Teil des Prothesenschaftes verbindbar sein. Der dritte Ausgang des wenigstens einen Kanals kann in diesem Fall an einer anderen Seite der Einrichtung angeordnet sein. Damit kann an dieser Stelle ein Verbindungselement, beispielsweise ein Schlauch, angeordnet werden, mit dem der dritte Ausgang des wenigstens einen Kanals mit der Durchgangsbohrung im Prothesenschaft verbunden werden kann. Dies ist insbesondere für den Fall von Vorteil, dass die im Prothesenschaft vorgesehene Durchgangsbohrung nicht an einer Stelle angeordnet ist, die im verbundenen Zustand der Einrichtung zugewandt ist. Die Einrichtung ist auf diese Weise flexibel für unterschiedlichste Ausgestaltungen von Prothesenschäften verwendbar.

Durch das Abschlusselement, dass mit dem dritten Ausgang verbindbar ist und in diesem Zustand ein Einströmen verhindert, wird gewährleistet, dass auch für den Fall, dass der erste Ausgang mit der Durchgangsbohrung im Prothesenschaft verbunden wird, ein Vakuum innerhalb des Prothesenschaftes zwischen Prothesenschaft und Amputationsstumpf des Patienten erzeugt werden kann, ohne dass zusätzliche Luft durch den dritten Ausgang des wenigstens einen Kanals einströmt.

Als besonders vorteilhaft hat sich herausgestellt, wenn das Abschlusselement ein Ein-Wege-Ventil aufweist, das in dem verbundenen Zustand des Abschlusselementes mit dem dritten Ausgang nur ein Ausströmen durch den dritten Ausgang aus dem wenigstens einen Kanal zulässt. Beim Anlegen einer Prothese mit einem Prothesenschaft, bei dem die Pumpe zum Erzeugen des Vakuums über eine derartige Einrichtung mit der Durchgangsbohrung im Prothesenschaft verbunden ist, kann im Prothesenschaft vorhandene Luft folglich zunächst durch das Ein-Wege-Ventil den Prothesenschaft verlassen. Dazu muss folglich nicht die Pumpe verwendet werden, so dass einerseits Energie eingespart und somit die Laufzeit der Pumpe verlängert werden kann und zudem eine unangenehme und störende Geräuschbelastung durch eine anspringende Pumpe verhindert wird. Zudem können kleinere Leckagen, die zu einem Einströmen von Luft in den Bereich zwischen Prothesenschaft und Amputationsstumpf führen, dadurch kompensiert werden, dass diese Luft allein durch beispielsweise eine Gehbewegung des Patienten durch das Ein-Wege-Ventil im Abschlusselement das Prothesensystem verlässt, ohne dass hierfür die Pumpe anspringen, verwendet oder abgenommen werden müsste.

Eine Einrichtung gemäß einem Ausführungsbeispiel der vorliegenden Erfindung muss lediglich so ausgebildet sein, dass sie eine in einem Prothesenschaft vorgesehen Durchgangsbohrung mit einer Pumpe verbinden kann. Wird sie als Adapterelement ausgebildet, kann die Einrichtung daher auch in herkömmliche Prothesensysteme nach dem Stand der Technik eingesetzt werden, die somit einfach nachrüstbar sind. Bevorzugt wird dabei ein System mit einem Adapterelement-der vorbeschriebenen Art sowie einer Pumpe mit einem Pumpengehäuse vorgesehen, wobei an dem Pumpengehäuse und an dem Adapterelement zueinander korrespondierende Verbindungsmittel ausgebildet sind, durch die das Adapterelement mit dem Pumpengehäuse verbindbar sind.

Auf diese Weise ist besonders einfach gewährleistet, dass zum einen immer eine passende Pumpe für das jeweilige Adapterelement vorgesehen ist, und das zum anderen eine sichere und einfache Verbindung der beiden Bauteile miteinander möglich ist. Dazu können besonders bevorzugt diese Verbindungsmittel beispielsweise als wenigstens eine Nut und wenigstens eine zu der Nut korrespondierende Feder ausgebildet sein. Dabei sind die Nut oder die Feder am Adapterelement und das jeweils andere Verbindungsmittel am Pumpengehäuse angeordnet. Auf diese Weise können das Adapterelement und das Pumpengehäuse einfach ineinander geschoben werden, so dass es hier zu einer sicheren Verbindung kommt. Natürlich ist diese Art der Befestigung der Pumpe an der Einrichtung auch dann von Vorteil, wenn die Einrichtung nicht als separates Adapterelement ausgebildet, sondern in ein anderes Bauteil des Prothesensystems integriert ist.

Bevorzugt umfassen die Verbindungsmittel eine an der Einrichtung vorgesehene Verbindungsplatte. Diese ist beispielsweise mit Elementen versehen, in die das Pumpengehäuse einrasten oder einschnappen kann. Alternativ oder zusätzlich dazu kann auch ein separates Befestigungselement vorgesehen sein, das bei Bedarf an der Verbindungsplatte befestigt werden kann. An diesem Befestigungselement ist dann die Pumpe befestigbar. Diese Ausgestaltung hat den Vorteil, dass etwaige an der Verbindungsplatte angeordnete Vorsprünge, die für die Anordnung der Pumpe direkt an der Verbindungsplatte notwendig sein können, nicht mehr vorhanden sein müssen und daher vom Träger des Prothesensystems nicht mehr versehentlich abgebrochen oder auf sonstige Weise beschädigt werden können.

Die Pumpe kann auch unlösbar beispielsweise mit der Verbindungsplatte verbunden, beispielsweise an diese anlaminiert, sein. Die Verbindungsplatte kann an der Einrichtung zum Beispiel durch Schnapp- oder Rastelement oder auch durch eine Schraubverbindung befestigt sein.

Vorzugsweise ist an dem Pumpengehäuse ein nach außen hervorstehender Pin angeordnet, der derart ausgebildet ist, dass der die Membran durchstößt, wenn das Pumpengehäuse mit der Einrichtung verbunden ist. Wird nun also das Pumpengehäuse mit der Einrichtung verbunden, dringt der Pin in den zweiten Ausgang des Kanals der Einrichtung ein. Dabei durchstößt er die Membran, die das hinter dem zweiten Ausgang angeordnete Ventil aufweist. Auf diese Weise wird das Ventil vom ersten Modus in den zweiten Modus gebracht, so dass nun die Pumpe sowohl Luft durch den zumindest einen Kanal der Einrichtung saugen als auch durch ihn hindurch pumpen kann. Alternativ ist der Pin so ausgestaltet, dass er auf die Außenflächen der als Schnabel oder Dom ausgebildeten Membran drückt und so die Membran öffnet. Dabei wird der Druck bevorzugt in Randbereichen der Membran aufgebracht, so dass sich im mittleren Bereich, in dem eine Schlitzung vorgesehen ist, eine Öffnung ergibt.

Um eine sichere Verbindung des Pumpengehäuses mit der Einrichtung zu gewährleisten, ist vorzugsweise sowohl am Pumpengehäuse als auch an der Einrichtung ein Kraftaufbringelement angeordnet, durch die eine Kraft hervorgerufen wird, wenn das Pumpengehäuse mit der Einrichtung verbunden ist. Diese Kraft ist dabei dem Lösen des Pumpengehäuses von der Einrichtung entgegengerichtet. Als besonders einfache Ausgestaltung derartiger Kraftaufbringelemente können beispielsweise Magnetelemente vorgesehen sein, die ungleichnamig gepolt sind und sich somit gegenseitig anziehen. Ein versehentliches Lösen des Pumpengehäuses von der Einrichtung während des Pumpvorganges ist somit zumindest erschwert. Alternativ oder zusätzlich können Rast- und/oder Schnappelemente vorgesehen sein, durch die ein versehentliches Lösen des Pumpengehäuses weiter erschwert wird.

In einer bevorzugten Ausgestaltung ist sowohl am Pumpengehäuse als auch an der Einrichtung jeweils wenigstens eine Einschubbohrung angeordnet, die im verbundenen Zustand miteinander fluchten, so dass ein Steckelement durch die Einschubbohrungen führbar ist. Auf diese Weise wird eine weitere Verbindung, die insbesondere formschlüssig ausgebildet sein kann, zwischen der Einrichtung und dem Pumpengehäuse erreicht, durch die einerseits erreicht wird, dass die Einrichtung und das Pumpengehäuse in einer optimalen Positionierung relativ zueinander angeordnet sind, und die andererseits auch für den Benutzer eines entsprechenden Prothesensystems leicht zu lösen ist. Bevorzugt ist das Steckelement beispielsweise in Form eines Steckschlüssels ausgebildet, der zum Lösen oder Anziehen von Schrauben an dem Prothesensystem verwendet werden kann. Auf diese Weise ist es beispielsweise besonders einfach möglich, etwa die Abdeckung eines Batteriefaches mit Schrauben zu sichern, so dass ein versehentliches Öffnen vermieden wird, und gleichzeitig dafür zu sorgen, dass das zum Öffnen des Batteriefaches benötigte Werkzeug am Prothesensystem getragen wird und gleichzeitig eine stabilisierende und sichernde Funktion erfüllt.

Vorzugsweise umfasst ein erfindungsgemäßes Prothesensystem einen Prothesenschaft, eine Einrichtung und einen Prothesenaufbau. Besonders vorteilhafterweise umfasst es zudem eine Pumpe mit einem oben beschriebenen Pumpengehäuse.

Durch eine Einrichtung gemäß den Ausführungsbeispielen der vorliegenden Erfindung ist es folglich möglich, eine aktive Unterdruckpumpe und eine Überdruckpumpe mit dem Prothesensystem zu verbinden und so das Absaugen bzw. das Hineinpumpen von Luft aus dem oder in das Innenvolumen zu ermöglichen. Das Ventil an der Einrichtung fungiert dabei im ersten Modus als passives Ausstoßventil, durch das sich unerwünschterweise im Innenvolumen befindende Luft ausgestoßen werden kann, während es im zweiten Modus als Zugang der jeweils angeordneten Pumpe zum Innenvolumen dient. Durch die vorteilhafte Ausgestaltung des Pumpengehäuses so, dass es direkt mit der Einrichtung verbindbar ist, ist es anders als im Stand der Technik nicht mehr nötig, einen separaten Schlauch vorzusehen, der das Prothesensystem bzw. die Einrichtung mit einer separaten Pumpe verbindet. Dadurch wird sowohl der apparative Aufwand verringert als auch das Anschließen der Pumpe an das Prothesensystem bzw. an die Einrichtung vereinfacht.

Das Ventil kann auch durch einen beispielsweise manuell zu betätigenden Schalter oder eine andere Anordnung aus dem ersten Modus in den zweiten Modus und umgekehrt bringbar sein.

Mit Hilfe einer Zeichnung wir nachfolgend ein Ausführungsbeispiel der vorliegenden Erfindung näher erläutert. Es zeigt:
- Figur 1 -: eine schematische Ansicht einer Einrichtung gemäß einem ersten Ausführungsbeispiel der vorliegenden Erfindung,
- Figur 2 -: einen schematischen Schnitt durch einen Teil eines Prothesensystems gemäß einem Ausführungsbeispiel der vorliegenden Erfindung,
- Figur 3 -: ein Adapterelement gemäß einem weiteren Ausführungsbeispiel der vorliegenden Erfindung beim Einschieben in ein Pumpengehäuse,
- Figur 4 -: die Darstellung aus Figur 3 aus einem anderen Blickwinkel,
- Figur 5 -: den schematischen Schnitt durch ein Adapterelement gemäß einem Ausführungsbeispiel der vorliegenden Erfindung mit dem Ventil im ersten Modus,
- Figur 6 -: die Darstellung aus Figur 5 mit dem Ventil im zweiten Modus,
- Figur 7 -: einen schematischen Schnitt durch einen Teil eines Prothesensystems gemäß einem weiteren Ausführungsbeispiel der vorliegenden Erfindung,
- Figur 8 -: die schematische Darstellung eines Domventils mit einer ersten Pinform,
- Figur 9 -: die schematische Darstellung eines Domventils mit einer zweiten Pinform,
- Figur 10 -: das Domventil aus Figur 9 aus einem weiteren Blickwinkel,
- Figur 11 -: das Domventil aus Figur 9 und 10 in einer schematischen Schnittdarstellung,
- Figur 12 -: eine schematische Darstellung eines Prothesensystems gemäß einem Ausführungsbeispiel der vorliegenden Erfindung,
- Figur 13 -: die Darstellung aus Figur 11 in einer Seitenansicht,
- Figur 14 -: die schematische Ansicht eines Prothesensystems gemäß einem weiteren Ausführungsbeispiel der vorliegenden Erfindung,
- Figur 15 -: die Darstellung aus den Figuren 11 und 12 in einer Schnittdarstellung und
- Figur 16 -: ein vergrößerten Ausschnitt aus Figur 14,
- Figur 17 -: eine schematische Darstellung eines Ausschnittes aus einem Prothesensystem gemäß einem weiteren Ausführungsbeispiel der vorliegenden Erfindung,
- Figur 18 -: ein Abschlusselement für eine Einrichtung gemäß einem weiteren Ausführungsbeispiel der vorliegenden Erfindung in einer Schnittdarstellung,
- Figur 19 -: eine schematische Schnittdarstellung durch einen Ausschnitt eines Prothesensystems gemäß einem weiteren Ausführungsbeispiel der vorliegenden Erfindung,
- Figur 20 -: einen vergrößerten Ausschnitt aus Figur 19,
- Figur 21 -: den Ausschnitt aus Figur 20 mit einem anderen Abschlusselement in der Einrichtung und
- Figur 22 -: den Ausschnitt aus den Figuren 20 und 21 mit einem weiteren Abschlusselement,
- Figur 23 -: die schematische Darstellung einer Einrichtung gemäß einem weiteren Ausführungsbeispiel der vorliegenden Erfindung,
- Figur 24 -: die schematische Darstellung der Einrichtung aus Figur 23 mit einem Pumpengehäuse und
- Figur 25 -: eine schematische Schnittdarstellung durch einen Ausschnitt aus Figur 24.

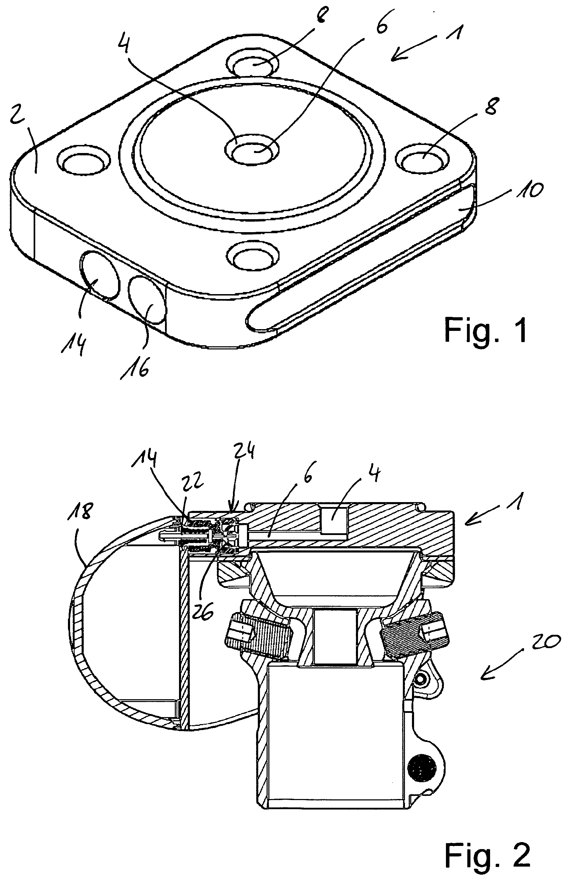

Figur 1 zeigt die schematische Ansicht einer Einrichtung gemäß einem ersten Ausführungsbeispiel der vorliegenden Erfindung, wobei die Einrichtung in Form eines Adapterelementes 1 ausgebildet ist. Auch wenn nachfolgend die Einrichtung immer als Adapterelement 1 dargestellt ist, gelten die Ausführungen auch für andere Ausgestaltungen einer Einrichtung gemäß eines Ausführungsbeispiels der vorliegenden Erfindung.

Das Adapterelement 1 weist eine Oberseite 2 auf, an der ein Prothesenschaft angeordnet werden kann, der in Figur 1 nicht dargestellt ist. In Figur 1 weist die Oberseite 2 einen ersten Ausgang 4 eines wenigstens einen Kanals 6 auf. Dieser erste Ausgang 4 kann mit einer am nicht gezeigten Prothesenschaft vorgesehenen Auslassöffnung verbunden werden. Man erkennt in Figur 1 mehrere Bohrungen 8, durch die Verbindungselemente führbar sind, mit denen das Adapterelement 1 am Prothesenschaft befestigbar ist. An einer Seitenfläche verfügt das Adapterelement 1 über eine Nut 10. An der dieser Seitenfläche gegenüberliegenden Seitenfläche, die in der in Figur 1 gezeigten Darstellung nicht zu sehen ist, befindet sich eine identische Nut 10. Diese beiden Nuten sind Teil von Verbindungsmitteln 12, durch die das Adapterelement 1 mit einem Pumpengehäuse 18, das in Figur 1 nicht dargestellt ist, verbindbar ist.

In Figur 1 ist zudem ein zweiter Ausgang 14 des Kanals 6 zu erkennen. An dem zweiten Ausgang 14 ist eine in Figur 1 nicht gezeigte Pumpe anschließbar, durch die Luft aus einem Innenvolumen, das zwischen einem Prothesenschaft und einem Liner bzw. einem Amputationsstumpf gebildet wird, heraus gepumpt werden kann. Da das sich im Inneren des Adapterelements 1 befindende Ventil in diesem Fall im zweiten Modus betrieben wird, kann auch Luft in dieses Innenvolumen hineingepumpt werden.

Neben dem zweiten Ausgang 14 ist ein erstes Kraftaufbringelement 16 dargestellt, das beispielsweise in Form eines Magneten ausgebildet sein kann.

Figur 2 zeigt einen Schnitt durch einen Teil eines Prothesensystems in einer schematischen Darstellung. Man erkennt das Adapterelement 1 mit dem Kanal 6, dem ersten Ausgang 4 und dem zweiten Ausgang 14. Am zweiten Ausgang 14 ist eine Pumpe mit einem Pumpengehäuse 18 angeordnet. In Figur 2 unterhalb des Adapterelementes 1 befindet sich ein Prothesenaufbau 20, auf den hier nicht weiter eingegangen werden soll.

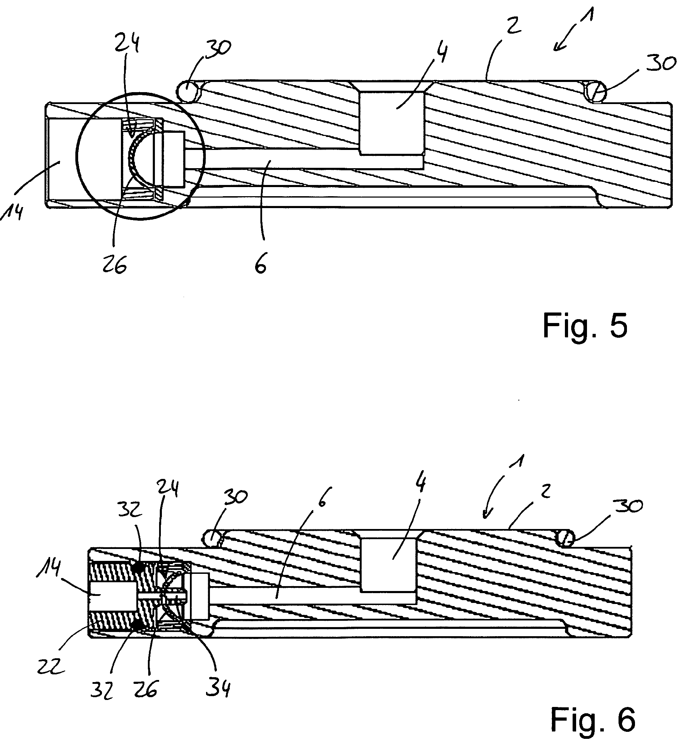

Am Pumpengehäuse 18 ist ein Pin 22 vorgesehen, der beim Verbinden des Adapterelementes 1 mit dem Pumpengehäuse 18 in den Ausgang 14 des Kanals 6 eindringt. Innerhalb des Kanals befindet sich ein Ventil 24, das über eine Membran 26 verfügt, die von dem Pin 22 durchstoßen wird. Dies wird mit Bezug auf Figur 6 näher erläutert werden.

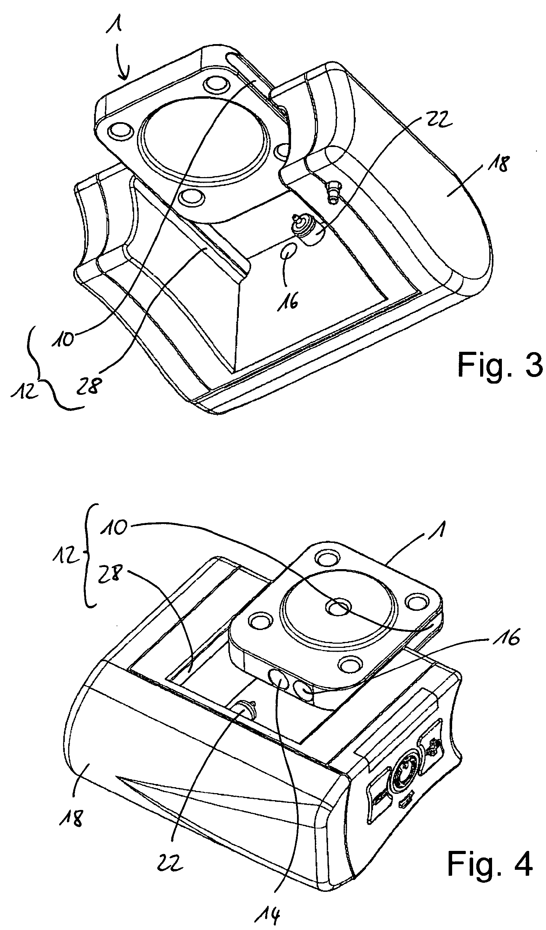

In den Figuren 3 und 4 ist gezeigt, wie ein Adapterelement 1 mit dem Pumpengehäuse 18 verbunden wird. Dabei zeigt Figur 3 eine schematische Ansicht von schräg unten, während Figur 4 eine schematische Ansicht von schräg oben zeigt. Am Pumpengehäuse 18 sind Federelemente 28 vorgesehen, die in die dafür am Adapterelement 1 vorgesehen Nuten 10 eingreifen. Nuten 10 und Federelemente 28 bilden zusammen die Verbindungsmittel 12. Auf diese Weise ist eine besonders einfache Verbindung zwischen Adapterelement 1 und Pumpengehäuse 18 möglich und zudem wird ein versehentliches Entfernen des Pumpengehäuses 18 während des Pumpvorganges deutlich erschwert.

In einer Seitenwandung des Pumpengehäuses 18 befindet sich der Pin 22. Wie in Figur 4 deutlich zu erkennen ist, dringt dieser Pin 22 in den zweiten Ausgang 14 des Kanals 6 ein, wo er eine in den Figuren 3 und 4 nicht gezeigte Membran 26 des Ventils 24 durchstößt und das Ventil so vom ersten in den zweiten Modus bringt. Alternativ dazu kann der Pin 22 auch auf eine andere Weise für eine Öffnung der Membran sorgen.

Sowohl am Adapterelement 1 als auch am Pumpengehäuse 18 ist jeweils ein Kraftaufbringelement 16 vorgesehen, die in diesem Fall beide als Magnetelemente ausgebildet sind. Wird nun das Adapterelement 1 mit dem Pumpengehäuse 18 verbunden, werden die beiden Kraftaufbringelemente 16 nah beieinander angeordnet oder sind sogar miteinander in Kontakt. Da es sich bei den beiden Kraftaufbringelementen 16 um unterschiedlich gepolte Magnetelemente handelt, ziehen sich die beiden an, so dass sie eine Kraft aufbringen, die einem Lösen des Adapterelementes 1 vom Pumpengehäuse 18 entgegengerichtet ist. Auf diese Weise wird ein versehentliches Entfernen beispielsweise während des Pumpvorganges weiter erschwert.

Figur 5 zeigt eine schematische Schnittdarstellung durch ein Adapterelement 1, während sich das Ventil 24, das in Figur 5 eingekreist dargestellt ist, im ersten Modus befindet. Das Adapterelement 1 verfügt über den Kanal 6 der den in Figur 5 nach oben gerichteten ersten Ausgang 4 und den in Figur 5 nach links gerichteten Ausgang 14 aufweist. An der Oberseite 2 des Adapterelementes 1 ist ein Dichtring 30 angeordnet, durch den eine Verbindung des Adapterelementes 1 zu dem an dieser Stelle anzuordnenden Prothesenschaft gesichert werden soll.

Das Ventil 24 verfügt über die Membran 26, die im in Figur 5 gezeigten Ausführungsbeispiel domförmig ausgebildet ist. In diesem Modus handelt es sich bei dem Ventil 24 um ein Ein-Wege-Ventil. Ein Durchströmen des Ventils 24 ist nur in einer Richtung vom ersten Ausgang 4 zum zweiten Ausgang 14 des Kanals 6 möglich. Die Membran 26 verfügt beispielsweise über eine Schlitzung, die ein derartiges Durchströmen ermöglicht.

Diese Ausgestaltung weist mehrere Vorteile auf. Zum einen ist es nicht möglich, versehentlich das Ventil 24 derart zu öffnen, dass Luft durch den Kanal 6 in Richtung auf den ersten Ausgang 4 strömt. Wird das Adapterelement 1 in diesem Zustand, in dem sich das Ventil 24 im ersten Modus befindet, an einem Prothesenschaft angeordnet, wird so gewährleistet, dass das Innenvolumen, das sich zwischen Prothesenschaft und Liner bzw. Amputationsstumpf befindet, nicht mit Luft gefüllt werden kann. Kommt es dennoch aufgrund von Bewegungen oder Undichtigkeiten zu einem Eindringen von Luft in dieses Innenvolumen wird das Innenvolumen beim Belasten der Prothese zusammengedrückt. Die darin enthaltene Luft kann durch den ersten Ausgang 4 in den Kanal 6 eindringen und durch das als Ein-Wege-Ventil wirkende Ventil 24 das Adapterelement 1 wieder verlassen. Damit ist gewährleistet, dass der zur stabilen Befestigung des Prothesensystems am Amputationsstumpf nötige Unterdruck aufrechterhalten bleibt.

Figur 6 zeigt die gleiche Situation, bei der sich jedoch das Ventil 24 im zweiten Modus befindet. Man erkennt wieder, dass das Ventil 24 über eine Membran 26 verfügt. Diese wird jedoch jetzt von dem in dem zweiten Ausgang 14 eingedrungenen Pin 22, der wie in den Figuren 2 bis 4 gezeigt, Teil des Pumpengehäuses 18 sein kann, durchstoßen. Zwischen dem Pin 22 und dem Adapterelement 1 befindet sich ein weiterer Dichtring 32, der eine dichte Verbindung zwischen dem Pin 22 und dem Adapterelement 1 gewährleistet.

In diesem Zustand befindet sich das Ventil 24 im zweiten Modus. Wie deutlich zu erkennen ist, weist der Pin 22 einen Durchgang 34 auf, durch den Luft vom Pumpengehäuse, das sich in Figur 6 links vom Pin 22 befindet, in den Kanal 6 eindringen kann.

Damit ist es zum einen möglich, durch eine an den zweiten Ausgang 14 angeschlossene Unterdruckpumpe Luft aus dem Kanal 6 des Adapterelementes 1 herauszupumpen. Dies ist insbesondere dann sinnvoll, wenn ein Innenvolumen, das sich zwischen einem an dem Adapterelement 1 befindlichen Prothesenschaft und dem Liner befindet, evakuiert werden soll. Zum anderen ist es in diesem Modul des Ventils 24 auch möglich, durch eine angeschlossene Überdruckpumpe Luft in den Kanal 4 hineinzupumpen. Dies ist insbesondere dann sinnvoll, wenn das Prothesensystem vom Träger abgelegt werden soll. Auf diese Weise wird das Innenvolumen zwischen dem nicht gezeigten Prothesenschaft und dem Amputationsstumpf mit Luft gefüllt, so dass die durch den Unterdruck hervorgerufene Haftwirkung aufgehoben wird. Die Membran 26 des Ventils 24 kann beispielsweise aus einem Elastomer, wie einem Fluorelastomer oder Silikon, bestehen. Das Ventil 24 ist vorteilhafterweise ein Schlitz- oder Kreuzschlitzventil oder ein Domventil. Wichtig ist lediglich, dass die Membran 26 vom Pin 22 durchstoßen oder durch Druck geöffnet werden kann, damit das Ventil 24 im zweiten Modus betrieben wird, und dass die Membran 26 wieder funktionsfähig schließt, wenn der Pin 22 aus der Membran 26 entfernt wird. Auf diese Weise ist es möglich, das Ventil 24 im Adapterelement 1 als Ein-Wege-Ventil im ersten Modus und als Zwei-Wege-Ventil im zweiten Modus zu verwenden. Dadurch, dass der Pin 22 Teil des Pumpengehäuses 18 ist, wird beim Abziehen der Pumpe von dem Adapterelement 1 auch der Pin 22 aus der Membran 26 des Ventils 24 gezogen. Damit wird das Ventil 24 vom zweiten Modus wieder in den ersten Modus gebracht, so dass ein beispielsweise durch eine angeschlossene Unterdruckpumpe hervorgerufener Unterdruck im Innenvolumen zwischen Prothesenschaft und Liner bestehen bleibt.

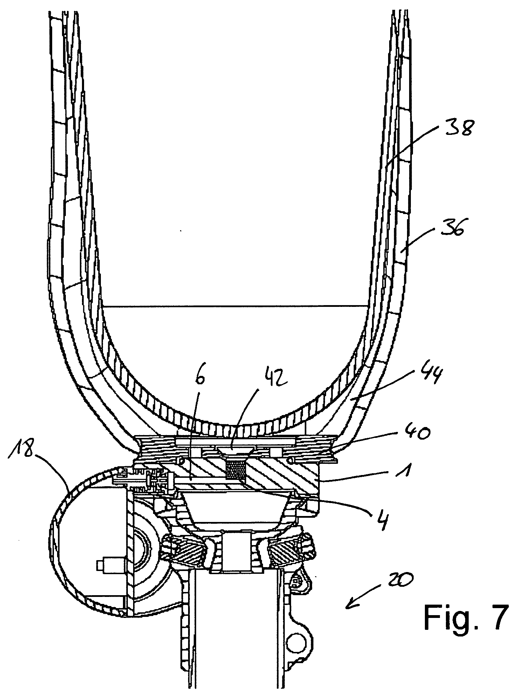

Figur 7 zeigt einen Ausschnitt aus einem Prothesensystem gemäß einem Ausführungsbeispiel der vorliegenden Erfindung. In einen Prothesenschaft 36, in dem sich ein Liner 38 befindet, wird ein Prothesenstumpf des Patienten eingesetzt. Unterhalb des Prothesenschaftes 36 befindet sich das Adapterelement 1 mit dem sich darin befindlichen Kanal 6. Das Adapterelement 1 ist über eine Befestigungsvorrichtung 40 am Prothesenschaft 36 angeordnet. In der Befestigungsvorrichtung 40 befindet sich eine Durchgangsbohrung 42, durch die der Kanal 6 des Adapterelementes 1 mit einem Innenvolumen 44 verbunden ist, das sich zwischen dem Liner 38 und dem Prothesenschaft 36 erstreckt.

Am Adapterelement 1, dessen ersten Ausgang 4 mit der Durchgangsbohrung 42 der Befestigungsvorrichtung 40 verbunden ist, ist das Pumpengehäuse 18 angeordnet, wie es bereits in Figur 2 dargestellt ist. In Figur 7 nach unten hin erstreckt sich der Protheseaufbau 20, auf den hier nicht weiter eingegangen werden soll.

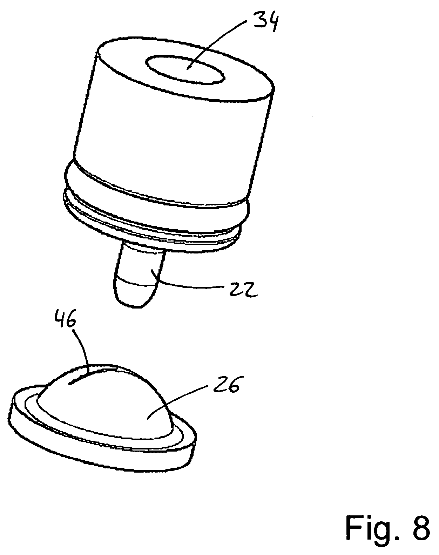

Figur 8 zeigt eine schematische Darstellung einer besonderen Ausgestaltung eines Domventils. Im unteren Teil ist die domförmig ausgebildete Membran 26 zu erkennen, die im in Figur 8 gezeigten Ausführungsbeispiel über einen Längsschlitz 46 verfügt. In Figur 8 oberhalb der Membran 26 ist der Pin 22 dargestellt, in dem sich der Durchgang 34 befindet. In der in Figur 8 dargestellten Situation ist ein Durchströmen der Membran 26 nur in einer Richtung von unten nach oben möglich. In diesem Fall öffnet sich der Längsschlitz 46 in der Membran 26 und lässt das strömende Medium im vorliegenden Fall also Luft, passieren. Ein Durchströmen der Membran 26 in der umgekehrten Richtung ist nicht möglich, da in diesem Fall der Längsschlitz 46 und damit die Membran 26 geschlossen bleiben. Wird das Ventil 24 in diesem Modus betrieben, besteht eine Druckdifferenz derart, dass vor dem Ventil 24, also auf der Seite des Pins 22, ein deutlich höherer Druck herrscht, als hinter dem Ventil 24, da das Ventil 24 auf dieser Seite mit dem Innenraum 44 verbunden ist, in dem ein Unterdruck herrscht. Durch diese Druckdifferenz werden die einzelnen Seiten der Membran 26, die durch den Längsschlitz 46 von einander getrennt sind, zusammengezogen und verschlossen, so dass in dieser Richtung keine Luft durch die Membran 26 hindurch gelangen kann.

Wird nun der Pin 22 im in Figur 8 gezeigten Ausführungsbeispiel nach unten bewegt, durchstößt er die Membran 26 und dringt durch den Längsschlitz 46 ein. Dadurch wird die Membran 26 geöffnet, so dass auch in einer Durchströmrichtung von oben nach unten durch den Durchgang 34 Luft geleitet werden kann.

Nachdem der Pin 22 wieder aus dem Längsschlitz 46 der Membran 26 herausgezogen wurde, schließt der Längsschlitz 46 wieder und ein Durchströmen der Membran 26 ist wieder nur in einer Richtung möglich.

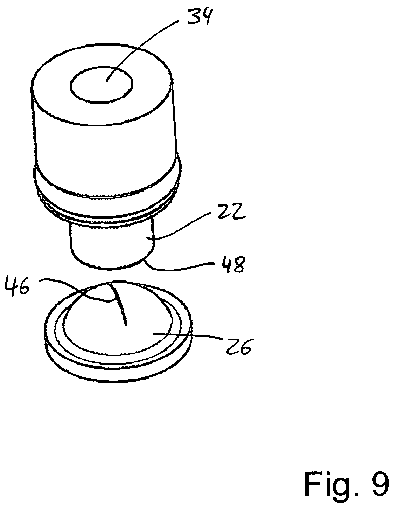

Figur 9 zeigt eine andere Ausgestaltung der in Figur 8 gezeigten Bauteile. Die Membran 26 verfügt wieder über einen Längsschlitz 46. Der Pin 22, durch den wieder der Durchgang 34 verläuft, durchstößt jedoch die Membran 26 nicht, sondern ist mit einer Druckkante 48 ausgestattet. Wird nun der Pin 22 auf die Membran 26 zu bewegt, durchstößt der Pin 22 die Membran 26 nicht, sondern übt durch die Druckkante 48 einen Druck auf die Membran 26 aus, wodurch ebenfalls der Längsschlitz 46 geöffnet wird und das Ventil 24, das die beiden Bauelemente beinhaltet, in Form eines Zwei-Wege-Ventils verwendet werden kann.

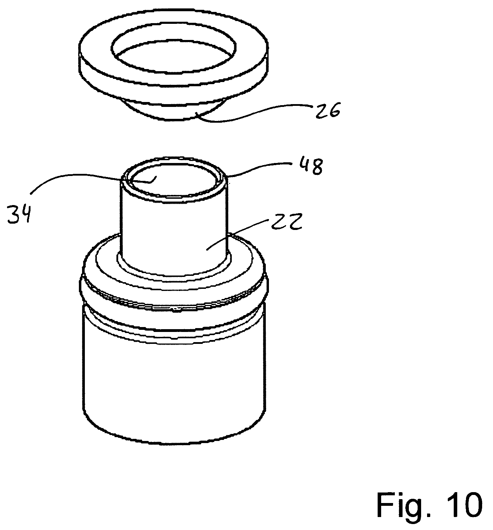

In Figur 10 sind die in Figur 9 gezeigten Bauteile aus einem anderen Blickwinkel gezeigt. Der Vorteil dieser Ausgestaltung gegenüber der in Figur 8 gezeigten Darstellung eines Pins 22 liegt darin, dass der Pin 22 im in den Figuren 9 und 10 gezeigten Ausführungsbeispiel den Längsschlitz 46 der Membran 26 nicht durchstoßen muss und ihn somit beim Durchstoßen und beim Herausziehen auch nicht beschädigen kann. Ein Ventil 24, das einen Pin 22 gemäß den Figuren 9 und 10 beinhaltet, ist daher weniger fehleranfällig und weist eine längere Lebensdauer auf.

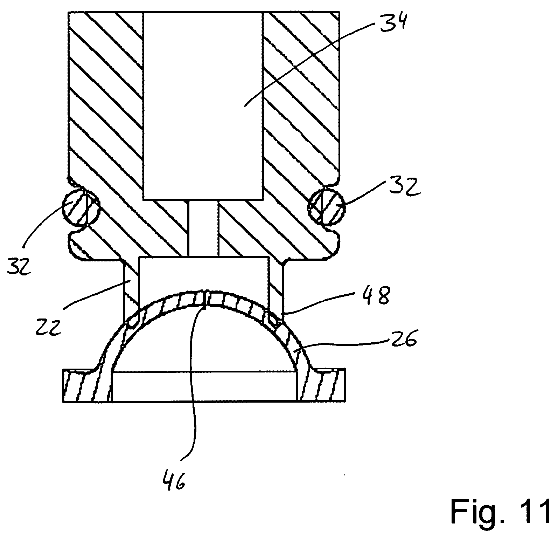

In Figur 11 ist die Situation dargestellt, bei der der Pin 22 gemäß den Figuren 9 und 10, an der Membran 26 des Ventils 24 anliegt. Dadurch öffnet sich der Längsschlitz 46 und das Ventil 24 kann als Zwei-Wegeventil verwendet werden.

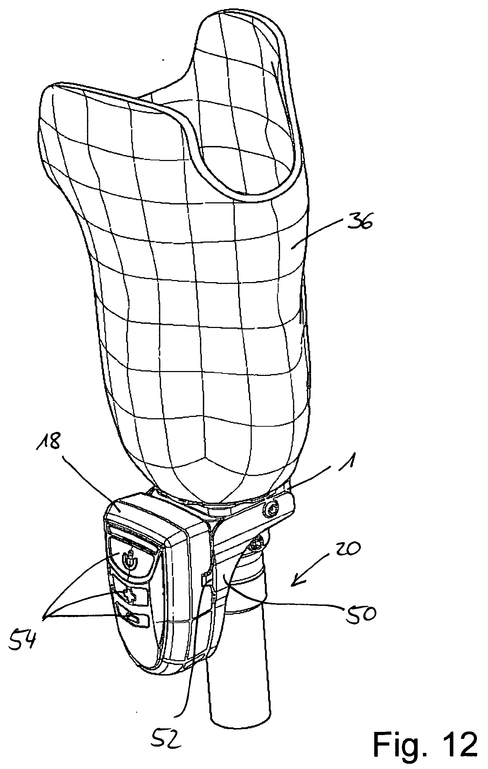

Figur 12 zeigt die schematische Darstellung eines Prothesensystems gemäß einem Ausführungsbeispiel der vorliegenden Erfindung. Im oberen Teil ist der Prothesenschaft 36 dargestellt, während sich am unteren Ende des Prothesensystems der Prothesenaufbau 20 erstreckt. Dazwischen befindet sich das Adapterelement 1, das im vorliegenden Ausführungsbeispiel durch eine an ihm befestigte Verbindungsplatte 50 nahezu vollständig verdeckt wird. Die Verbindungsplatte 50 verfügt über Rast- oder Schnappelemente 52, durch die das Pumpengehäuse 18 mit der Verbindungsplatte 50 verbindbar ist. An der Außenseite des Pumpengehäuses 18 sind verschiedene Bedienelement 50 vorgesehen, durch die die Funktion der Pumpe beeinflusst werden kann.

Durch das Vorsehen der separaten Verbindungsplatte 50 mit den sich daran befindlichen Rast- oder Schnappelementen 52 ist eine besonders einfache und dennoch sichere sowie platzsparende Möglichkeit gegeben, das Pumpengehäuse 18 zu befestigen.

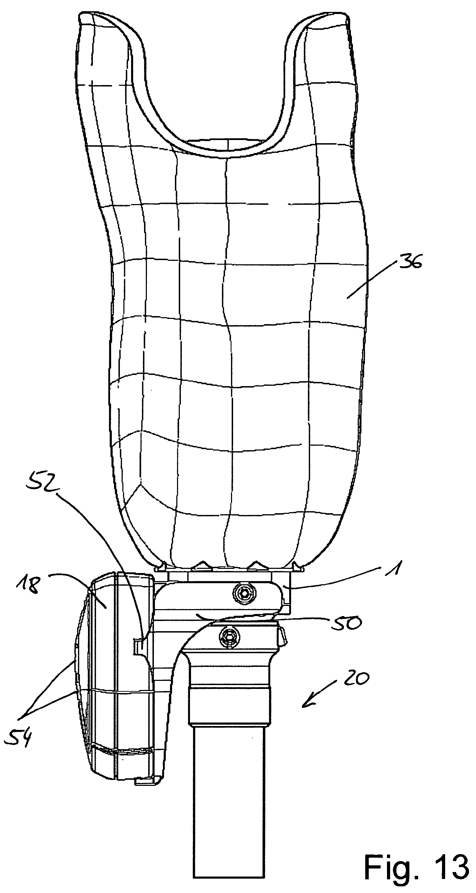

Figur 13 zeigt das Prothesensystem aus Figur 12 in einer Seitenansicht. Durch die spezielle Ausgestaltung der Verbindungsplatte 50 ist es möglich, das Pumpengehäuse 18 möglichst platzsparend und unauffällig zu befestigen und dennoch eine gute Erreichbarkeit durch den Träger des Prothesensystems zu gewährleisten.

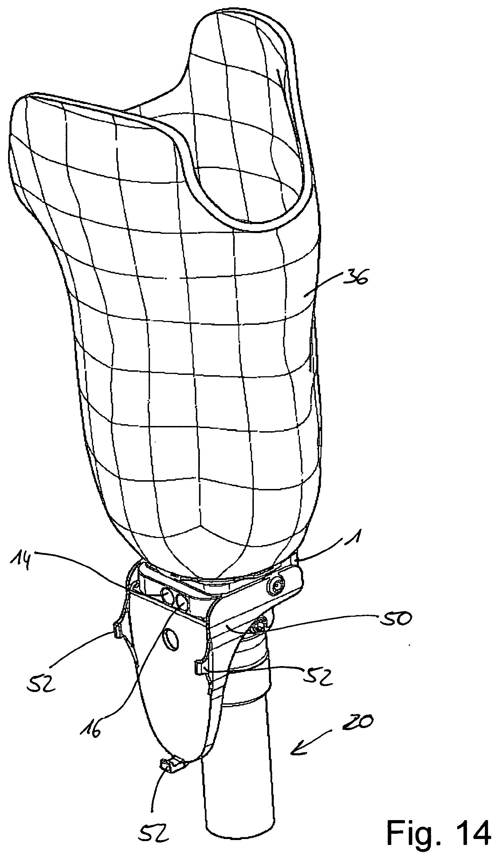

In Figur 14 ist das Prothesensystem aus den Figuren 12 und 13 gezeigt, bei dem das Pumpengehäuse 18 entfernt wurde. An der Seite, an der an die Verbindungsplatte 50 das Pumpengehäuse 18 anzuordnen ist, verfügt die Verbindungsplatte 50 im gezeigten Ausführungsbeispiel über drei Rast- oder Schnappelemente 52, durch die eine sichere und erschütterungsfeste Lagerung des Pumpengehäuses 18 an der Verbindungsplatte 50 gewährleistet ist. Zudem verfügt auch das Adapterelement 1 über ein Kraftaufbringelement 16, das, wie bereits beschrieben, beispielsweise in Form eines Magneten ausgestaltet sein kann.

Neben diesem Kraftaufbringelement ist der zweite Ausgang 14 des Adapterelementes 1 dargestellt, an den die nicht gezeigte Pumpe angeschlossen wird.

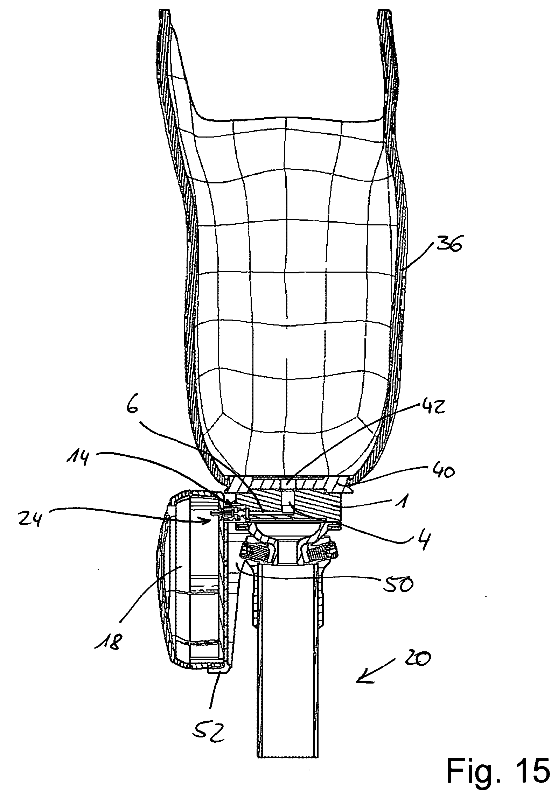

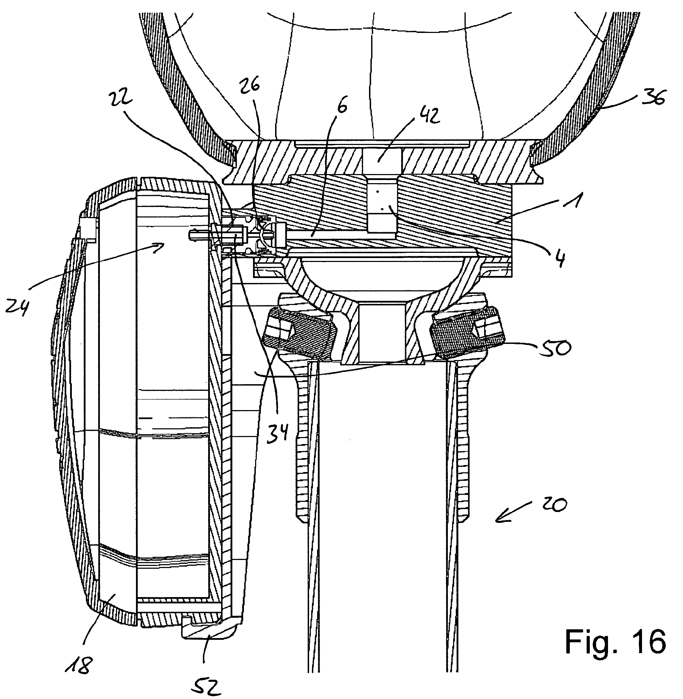

Figur 15 zeigt die Darstellung aus den Figuren 12 und 13 in einer Schnittdarstellung. Am unteren Ende des Prothesenschaftes 36 befindet sich die Durchgangsbohrung 42, die über den ersten Ausgang 4 mit dem Kanal 6 des Adapterelementes 1 verbunden ist. Am zweiten Ausgang 14 des Kanals 6 ist das Ventil 24 dargestellt. Der mittlere Bereich der Figur 15 ist in Figur 16 vergrößert dargestellt.

Das Ventil 24 verfügt über die Membran 26, die beispielsweise über einen Längsschlitz 46, wie er beispielsweise in den Figuren 8 und 9 dargestellt ist, verfügt. Durch diesen Längsschlitz stößt in Figur 16 der Pin 22 mit dem sich darin befindlichen Durchgang 34. Dadurch wird das Ventil 24 in der in Figur 16 gezeigten Situation in Form eines Zwei-Wege-Ventils betrieben, so dass durch die Pumpe, die sich Pumpengehäuse 18 befindet, das Innenvolumen 44, das sich zwischen dem Prothesenschaft 36 und dem in Figur 16 nicht gezeigten Liner 38 befindet, evakuiert werden kann.

Nachdem ein ausreichend starker Unterdruck auf diese Weise hergestellt wurde, kann entweder die Pumpe selbst mit dem Pumpengehäuse 18 von der Verbindungsplatte 50 entfernt werden, wodurch der Pin 22 in der in Figur 16 dargestellten Ausführungsform nach links zurückschnappt. Dadurch durchdringt der Pin 22 nicht mehr die Membran 26 und das Ventil 24 wird nur noch in Form eines Ein-Wege-Ventils betrieben.

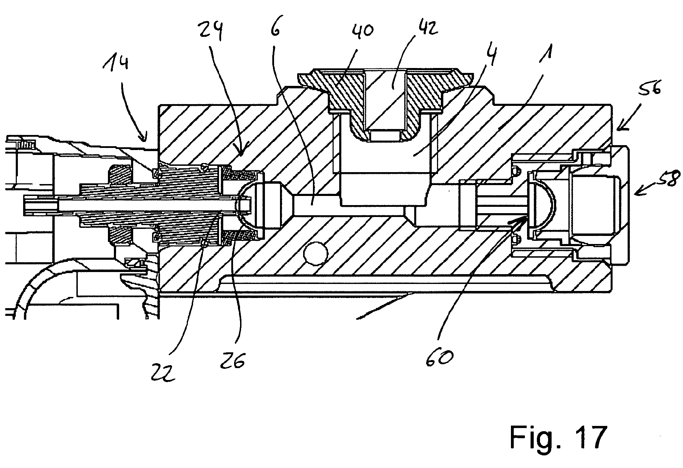

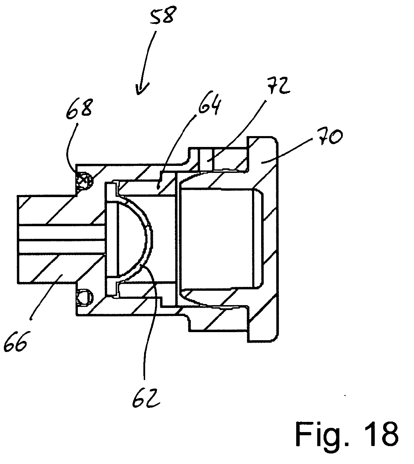

Figur 17 zeigt das Adapterelement 1 mit dem Kanal 6, dem ersten Ausgang 4 sowie im zweiten Ausgang 14, in dem sich das Ventil 24 mit der Membran 26 befindet, die vom Pin 22 der Pumpe durchstochen wird. Im ersten Ausgang 4 ist ein Dichtelement 82 angeordnet, das einen luftdichten Anschluss an die Befestigungsvorrichtung 40 eines nicht dargestellten Prothesenschaftes ermöglicht. In diesem befindet sich die Durchgangsbohrung 42. Das in Figur 17 dargestellte Adapterelement 1 verfügt zudem über einen dritten Ausgang 56, in dem sich ein Abschlusselement 58 befindet. Dieses verfügt über ein Ein-Wege-Ventil 60, das einen Luftaustritt nur im in Figur 17 dargestellten Ausführungsbeispiel nach rechts erlaubt. Das Abschlusselement 58 ist in Figur 18 detaillierter dargestellt. Man erkennt eine Ventilmembran 62, die einen Luftdurchtritt in der gezeigten Darstellung von links nach rechts erlaubt, von rechts nach links jedoch verhindert. Die Ventilmembran 62 liegt zwischen einer Ventilhalterung 64 und einem Ventilgehäuse 66 an. Im Ventilgehäuse 66 befindet sich ein Dichtring 68, der verhindert, dass Luft zwischen der Wandung des dritten Ausgangs 56 und dem Ventilgehäuse 66 hindurch tritt.

Im rechten Bereich des Abschlusselementes ist eine Schutzkappe 70 dargestellt, die das Ventil vor Verschmutzungen schützt. Im oberen Bereich des Ventilgehäuses 66 befindet sich eine Austrittsbohrung 72, durch die Gas, das durch das Ventil hindurch getreten ist, das Abschlusselement 58 an der Schutzkappe 70 vorbei verlassen kann.

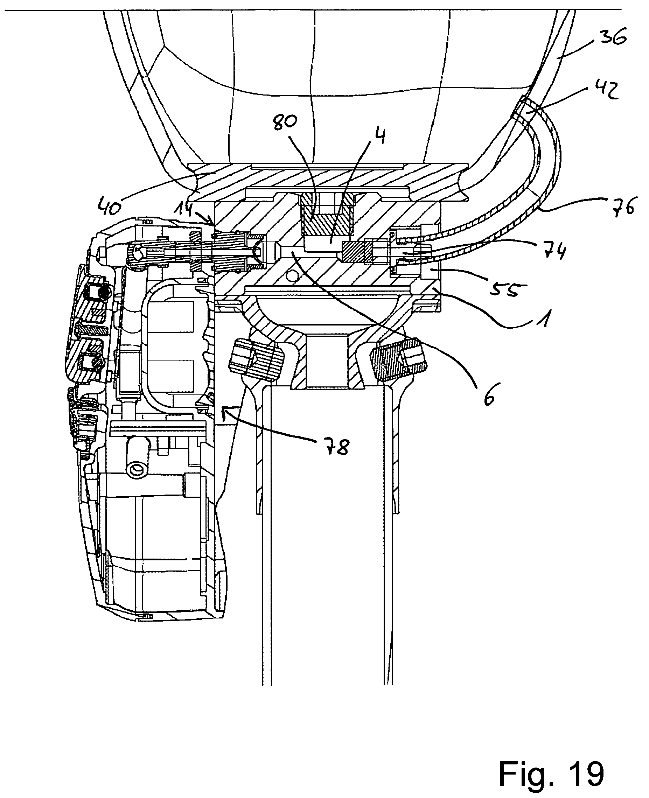

Figur 19 zeigt eine schematische Schnittdarstellung durch einen Ausschnitt eines Prothesensystems. Man erkennt das Adapterelement, das über den ersten Kanal 6 verfügt, der im gezeigten Ausführungsbeispiel den dritten Ausgang 56 aufweist. In diesem befindet sich jedoch nicht das Abschlusselement 58, wie es in Figur 18 dargestellt ist, sondern ein Schlauchanschluss 74, an dem ein Schlauch 76 angeordnet ist. Dieser ist mit der Durchgangsbohrung 42 im Prothesenschaft 36 verbunden. Durch eine angeordnete Pumpe 78, die mit dem zweiten Ausgang 14 verbunden ist, wird also Luft durch den Schlauchanschluss 74 und den Schlauch 76 aus dem Prothesenschaft 36 herausgepumpt. Der erste Ausgang 4 des Kanals 6 ist durch ein Verschlusselement 80 verschlossen. Die gezeigte Ausführungsform ist für den Fall von Interesse, wenn die Befestigungsvorrichtung 40 des Prothesenschaftes 36 an der dem ersten Ausgang 4 zugewandten Seite keine Durchgangsbohrung aufweist.

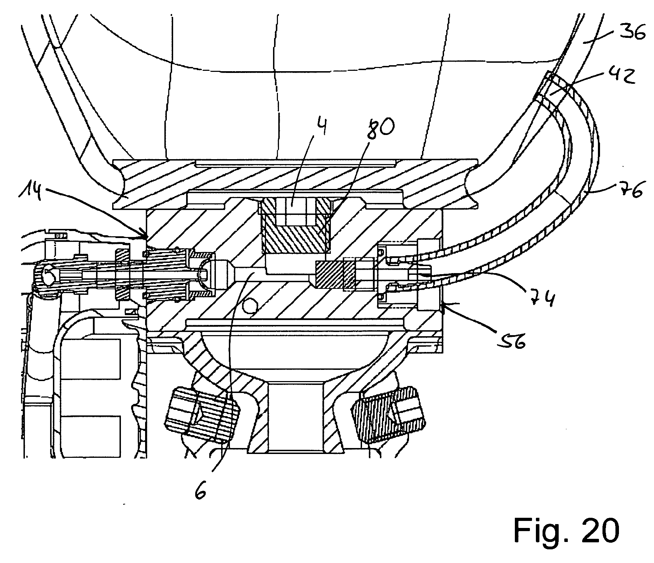

Figur 20 zeigt einen vergrößerten Ausschnitt aus Figur 19.

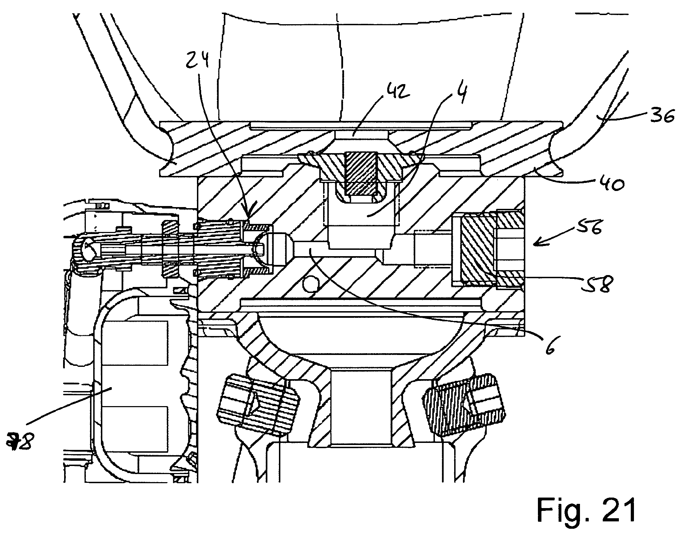

Figur 21 zeigt den gleichen Ausschnitt, wobei jedoch nun die Befestigungsvorrichtung 40 des Prothesenschaftes 36 an der dem ersten Ausgang 4 zugewandten Seite die Durchgangsbohrung 42 aufweist und über das Dichtelement 82 mit dem Adapterelement 1 verbunden ist. Die Pumpe 78 pumpt folglich durch das Ventil 24 Luft aus dem Kanal 6 heraus, die durch den ersten Ausgang 4 und die Durchgangsbohrung 42 aus dem Innenbereich des Prothesenschaftes 36 in den Kanal 6 eintritt. Der dritte Ausgang 56 ist im gezeigten Ausführungsbeispiel mit dem Abschlusselement 58 verschlossen. Dabei bildet das hier gezeigte Abschlusselement 58 lediglich einen Verschluss, so dass Luft weder durch den dritten Ausgang 56 in den Kanal 6 eindringen noch aus ihm austreten kann. Auf diese Weise wird die gleiche Funktion erreicht, wie sie in dem in Figur 15 dargestellten Ausführungsbeispiel gezeigt ist, bei der das Adapterelement 1 bzw. der wenigstens eine Kanal 6 nur einen ersten Ausgang 4 und einen zweiten Ausgang 14 aufweist.

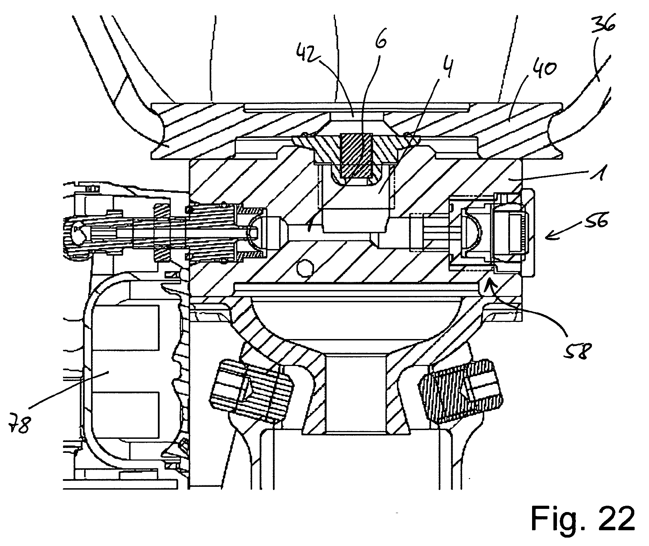

Figur 22 zeigt noch einmal einen entsprechenden Ausschnitt, bei dem das Adapterelement 1 mit der Befestigungsvorrichtung 40 des Prothesenschaftes 36 über das Dichtelement 82 verbunden ist. Die Durchgangsbohrung 42 befindet sich wieder in der Befestigungsvorrichtung 40 und somit an der dem ersten Ausgang 4 des Kanals 6 zugewandten Seite. Die Pumpe 78 pumpt also wie in dem in Figur 21 dargestellten Ausführungsbeispiel Luft aus dem Innenbereich des Prothesenschaftes 21 heraus. Im dritten Ausgang 56 befindet sich nun das Abschlusselement 58, das bereits in Figur 18 gezeigt ist. Luft aus dem Kanal 6 kann folglich durch das Abschlusselement 58 und den dritten Ausgang 56 den Kanal 6 verlassen. Dies ist insbesondere von Vorteil, wenn sich beispielsweise durch eine kleine Leckage Gas im Prothesenschaft 36 des Prothesensystems ansammelt. Durch eine Gehbewegung des Patienten wird dieses Gas durch die Durchgangsbohrung 42 in den Kanal 6 eingedrückt. Sofern die Pumpe 78 nicht in Betrieb ist, kann das Gas den Kanal durch den zweiten Ausgang 14 nicht verlassen. Stattdessen ist es möglich, das Gas aus dem Kanal 6 abzuführen und somit das Vakuum, das innerhalb des Prothesenschaftes gewünscht ist, um die Prothese am Amputationsstumpf des Patienten festzuhalten, aufrechterhalten werden kann, ohne dass die Pumpe 78 eingeschaltet werden muss. Dadurch kann Energie gespart, die Geräuschbelastung vermindert und zudem die Lebensdauer der Pumpe erhöht werden.

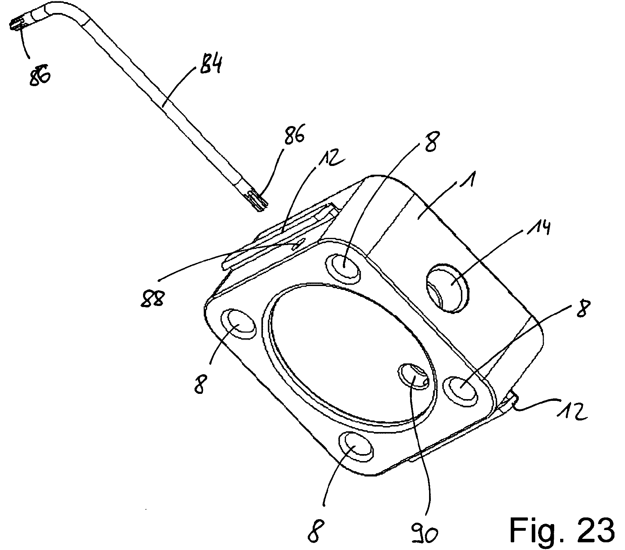

Figur 23 zeigt das Adapterelement 1 gemäß einem weiteren Ausführungsbeispiel der vorliegenden Erfindung. Es verfügt über vier Bohrungen 8 sowie Verbindungsmittel 12, die im in Figur 23 gezeigten Ausführungsbeispiel in Form von hakenförmigen Vorsprüngen, die sich über nahezu die gesamte Seitenlänge des Adapterelementes 1 erstrecken, ausgebildet sind. An einer Seitenfläche ist der zweite Ausgang 14 zu erkennen.

Neben dem Adapterelement 1 ist ein Steckelement 84 dargestellt, das an seinen beiden Enden über jeweils einen Steckschlüssel 86 verfügt. Das Steckelement 84 kann in eine dafür im Adapterelement 1 vorgesehene Einschubbohrung 88 eingeschoben werden.

Im Pumpengehäuse 18, das in Figur 23 nicht dargestellt ist, befindet sich eine weitere Einschubbohrung 88, die so angeordnet ist, dass die beiden Einschubbohrungen 88 am Adapterelement 1 und an dem Pumpengehäuse 18 miteinander fluchten. In diesem Zustand wird das Steckelement 84 in die Einschubbohrungen 88 eingeschoben und sorgt so für eine Verriegelung und Befestigung der beiden Bauteile aneinander.

In Figur 23 ist am Adapterelement 1 zudem eine Kugelbolzenbohrung 90 zu erkennen, in der ein in Figur 23 nicht gezeigter Kugelbolzen federbelastet geführt ist. Wird das Steckelement 84 in die Einschubbohrung 88 eingeführt, kommt es zu einer Verschiebung des Kugelbolzens gegen die ihn belastende Feder, so dass hier eine weitere Befestigung und Verstärkung der Verbindung erreicht wird.

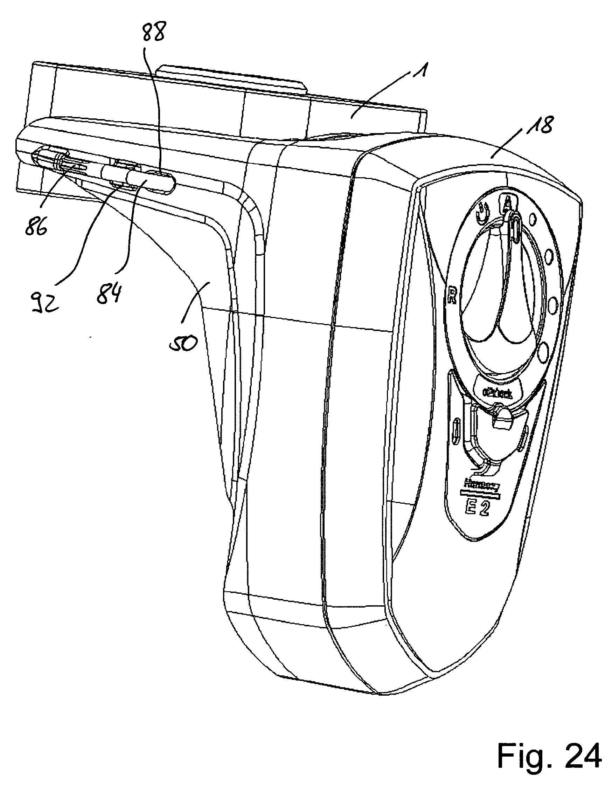

Figur 24 zeigt das Adapterelement 1, das mit dem Pumpengehäuse 18 über die bereits in Figur 13 dargestellte Verbindungsplatte 50 verbunden ist. Man erkennt einen Teil des Steckelementes 84, das in die Einschubbohrung 88 des Pumpengehäuses 18 sowie die in Figur 24 nicht zu erkennende Einschubbohrung 88 des Adapterelementes 1 eingeschoben ist. Der herausstehende Anteil des Steckelementes 84 ist in einem Clipelement 92 angeordnet und so gegen ein Herausrutschen aus den Einschubbohrungen 88 gesichert. Am Ende des Steckelementes ist der Steckschlüssel 86 dargestellt.

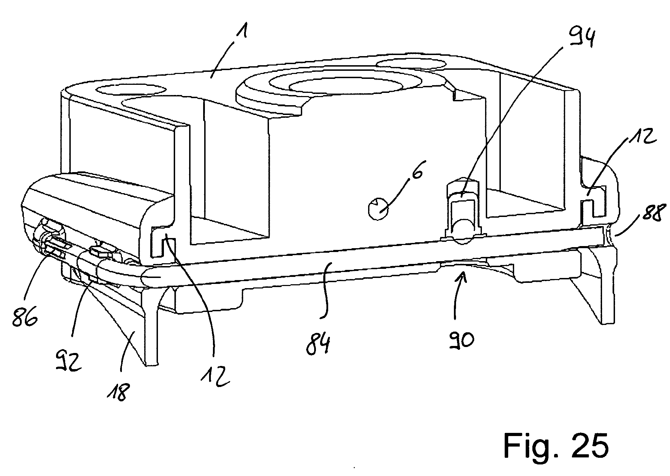

Figur 25 zeigt das Adapterelement 1 sowie einen Teil des Pumpengehäuses 18 in einer Schnittdarstellung. Man erkennt seitlich am Adapterelement 1 die Verbindungsmittel 12, die einen hakenförmigen Querschnitt aufweisen und in eine entsprechend geformte Nut am Pumpengehäuse 18 eingeschoben wurden. Der Schnitt der Schnittdarstellung von Figur 25 verläuft genau durch die Einschubbohrung 88, in der in Figur 25 das Steckelement 84 dargestellt ist. Das herausragende Ende ist wieder in dem Clipelement 92 angeordnet und gegen ein Herausrutschen gesichert.

Innerhalb der Kugelbolzenbohrungen 90 ist nun ein Kugelbolzen 94 dargestellt, der durch eine nicht gezeigte Feder in Figur 25 nach unten federbelastet ist. Durch das eingeschobene Steckelement 84 wird der Kugelbolzen 94 nach oben gegen die ihn belastende Feder gedrückt und übt nun folglich eine Kraft auf das Steckelement 84 auf, die einen sicheren Halt gewährleistet. Um diesen Effekt zu verstärken, kann in dem Bereich, in dem sich im eingeschobenen Zustand des Steckelementes 84 der Kugelbolzen 94 befindet, eine Verringerung des Querschnittes des Steckelementes 84 vorgesehen sein, so dass es hier zu einer weiteren Rastverbindung kommt. In der Schnittfläche des Adapterelementes 1 ist der Kanal 6 zu erkennen.

**Bezugszeichenliste**

| | | | |
|---|---|---|---|
| 1 | Adapterelement | 58 | Abschlusselement |
| 2 | Oberseite | 60 | Ein-Wege-Ventil |
| 4 | Erster Ausgang | 62 | Ventilmembran |
| 6 | Kanal | 64 | Ventilhalterung |
| 8 | Bohrung | 66 | Ventilgehäuse |
| 10 | Nut | 68 | Dichtring |
| 12 | Verbindungsmittel | 70 | Schutzkappe |
| 14 | Zweiter Ausgang | 72 | Austrittsbohrung |
| 16 | Kraftaufbringelement | 74 | Schlauchanschluss |
| 18 | Pumpengehäuse | 76 | Schlauch |
| 20 | Prothesenaufbau | 78 | Pumpe |
| 22 | Pin | 80 | Verschlusselement |
| 24 | Ventil | 82 | Dichtelement |
| 26 | Membran | 84 | Steckelement |
| 28 | Federelement | 86 | Steckschlüssel |
| 30 | Dichtring | 88 | Einschubbohrung |
| 32 | Dichtring | 90 | Kugelbolzenbohrung |
| 34 | Durchgang | 92 | Clipelement |
| 36 | Prothesenschaft | 94 | Kugelbolzen |
| 38 | Liner | | |
| 40 | Befestigungsvorrichtung | | |
| 42 | Durchgangsbohrung | | |
| 44 | Innenvolumen | | |
| 46 | Längsschlitz | | |
| 48 | Druckkante | | |
| 50 | Verbindungsplatte | | |
| 52 | Rast- oder Schnappelement | | |
| 54 | Bedienelement | | |
| 56 | Dritter Ausgang | | |

## Patentansprüche

1. Einrichtung zum Verbinden einer Pumpe mit einer in einem Prothesenschaft (36) vorgesehenen Durchgangsbohrung (42), wobei die Einrichtung zumindest einen Kanal (6) mit
einem ersten Ausgang (4) zum Verbinden mit der in dem Prothesenschaft (36) vorgesehenen Durchgangsbohrung (42) und einem zweiten Ausgang (14), der mit der Pumpe verbindbar ist, und
wenigstens ein Ventil (24) umfasst
wobei das Ventil (24) in einen ersten Modus und in einen zweiten Modus bringbar ist, wobei das Ventil (24) in dem ersten Modus ein Ein-Wege-Ventil, das nur einen Durchfluss von dem ersten Ausgang (4) zu dem zweiten Ausgang (14) erlaubt,
**dadurch gekennzeichnet, dass** das Ventil in dem zweiten Modus ein Zwei-Wege-Ventil ist, so dass Luft in beide Richtungen durch den Kanal strömen kann, wobei jeweils das Ventil durchströmt wird.

2. Einrichtung nach Anspruch 1, **dadurch gekennzeichnet, dass** das Ventil (24) eine Membran (26) umfasst und vom ersten Modus in den zweiten Modus bringbar ist, indem die Membran (26) durch ein Durchstoßelement (22) durchstoßen oder durch auf die Membran (26) aufgebrachten Druck geöffnet wird.

3. Einrichtung nach einem der vorstehenden Ansprüche **dadurch gekennzeichnet, dass** der zumindest eine Kanal (6) einen dritten Ausgang (56) zum Verbinden mit der in dem Prothesenschaft (36) vorgesehenen Durchgangsbohrung (42) und ein Abschlusselement (58) aufweist, dass mit dem dritten Ausgang (56) verbindbar ist und in einem verbundenen Zustand ein Einströmen durch den dritten Ausgang (56) in den wenigstens einen Kanal (6) verhindert.

4. Einrichtung nach Anspruch 3, **dadurch gekennzeichnet, dass** das Abschlusselement (58) ein Ein-Wege-Ventil (60) aufweist, das in dem verbundenen Zustand des Abschlusselementes (58) mit dem dritten Ausgang (56) nur ein Ausströmen durch den dritten Ausgang (56) aus dem wenigstens einen Kanal (6) zulässt.

5. System mit einer Einrichtung nach einem der vorstehenden Ansprüche und einer Pumpe mit einem Pumpengehäuse (18), wobei an dem Pumpengehäuse (18) und an der Einrichtung zueinander korrespondierende Verbindungsmittel (12) ausgebildet sind, durch die die Einrichtung mit dem Pumpengehäuse (18) verbindbar ist.

6. System nach Anspruch 5, **dadurch gekennzeichnet, dass** die Verbindungsmittel (12) als wenigstens eine Nut (10) und eine zu der Nut (10) korrespondierende Feder (28) ausgebildet sind.

7. System nach Anspruch 5, **dadurch gekennzeichnet, dass** die Verbindungsmittel (12) eine an der Einrichtung vorgesehene Verbindungsplatte (50) umfassen.

8. System nach Anspruch 5, 6 oder 7, **dadurch gekennzeichnet, dass** an dem Pumpengehäuse (18) ein nach außen hervorstehender Pin (22) angeordnet ist, der derart ausgebildet ist, dass er die Membran (26) durchstößt oder die Membran (26) durch auf die Membran (26) aufgebrachten Druck öffnet, wenn das Pumpengehäuse (18) mit der Einrichtung verbunden ist.

9. System nach einem der Ansprüche 5 bis 8, **dadurch gekennzeichnet, dass** am Pumpengehäuse (18) und an der Einrichtung jeweils ein Kraftaufbringelement (16) angeordnet ist, die gemeinsam eine Kraft hervorrufen, wenn das Pumpengehäuse (18) mit der Einrichtung verbunden ist, wobei die Kraft einem Lösen des Pumpengehäuses (18) von der Einrichtung entgegengerichtet ist.

10. System nach Anspruch 9, **dadurch gekennzeichnet, dass** am Pumpengehäuse (18) und an der Einrichtung jeweils eine Einschubbohrung (88) angeordnet ist, die in verbundenem Zustand miteinander fluchten, so dass ein Steckelement (84) durch die Einschubbohrung (88) führbar ist.

11. Prothesensystem mit einem Prothesenschaft, einer Einrichtung nach einem der Ansprüche 1 bis 4 und einem Prothesenaufbau (20).

12. Prothesensystem mit einem Prothesenschaft, einem System nach einem der Ansprüche 5 bis 10 und einem Prothesenaufbau (20).

## Claims

1. A device for connecting a pump to a through-bore (42) which is provided in a prosthesis socket (36), wherein the device includes
at least one channel (6) with
a first orifice (4) for connecting to the through-bore (42) provided in the prosthesis socket (36) and
a second orifice (14) which is connectable to the pump, and
at least one valve (24),
wherein the valve (24) is movable into a first mode and into a second mode,
wherein the valve (24) is a one-way valve in the first mode in which the valve only allows a flow in the direction from the first orifice (4) to the second orifice (14) **characterized in that** the valve is a two-way valve in the second mode such that air is able to flow in both directions through the channel (6), wherein in both modes air flows through the valve.

2. The device as claimed in claim 1, **characterized in that** the valve (24) includes a diaphragm (26) and is movable from the first mode into the second mode by the diaphragm (26) being pushed through by a push-through element (22) or by being opened by pressure applied to the diaphragm.

3. The device as claimed in one of the preceding claims, **characterized in that** the at least one channel (6) comprises a third orifice (56) for connecting to the through-bore (42) provided in the prosthesis socket (36) and an end element (58) which is connectable to the third orifice (56) and in a connected state prevents inflow through the third orifice (56) into the at least one channel (6).

4. The device as claimed in claim 3, **characterized in that** the end element (58) comprises a one-way valve (60) which, with the end element (58) connected to the third orifice (56), only allows outflow through the third orifice (56) out of the at least one channel (6).

5. A system having a device as claimed in one of the preceding claims and a pump with a pump housing (18), wherein connecting means (12), which correspond with one another and by way of which the device is connectable to the pump housing (18), are realized on the pump housing (18) and on the device.

6. The system as claimed in claim 5, **characterized in that** the connecting means (12) include at least one groove (10) and one tongue (28) which corresponds to the groove (10).

7. The system as claimed in claim 5, **characterized in that** the connecting means (12) include a connecting plate (50) which is provided on the device.

8. The system as claimed in claim 5, 6 or 7, **characterized in that** an outwardly projecting pin (22), which is configured in such a manner that it pushes through the diaphragm (26), is arranged on the pump housing (18) or the diaphragm (26) opens as a result of pressure applied to the diaphragm (26) when the pump housing (18) is connected to the device.

9. The system as claimed in one of claims 5 to 8, **characterized in that** a force-applying element (16) is arranged both on the pump housing (18) and on the device, said force-applying elements together producing a force when the pump housing (18) is connected to the device, wherein the force counters a releasing of the pump housing (18) from the device.

10. The system as claimed in claim 9, **characterized in that** an insert bore (88) is arranged both on the pump housing (18) and on the device, said insert bores being aligned with one another in the connected state such that a plug-in element (84) is guidable through the insert bore (88).

11. A prosthesis system having a prosthesis socket, a device as claimed in one of claims 1 to 4 and a prosthesis construction (20).

12. The prosthesis system having a prosthesis socket, a system as claimed in one of claims 5 to 10 and a prosthesis construction (20).

## Revendications

1. Agencement pour relier une pompe à un perçage traversant (42) ménagé dans une tige de prothèse (36), dans lequel le système inclut au moins un canal, avec
une première sortie (4) à relier au perçage traversant (42) ménagé dans la tige de prothèse (36), et
une seconde sortie (14) susceptible d'être reliée à la pompe,
et au moins une valve (24)
dans lequel la valve (24) est susceptible d'être amenée dans un premier mode et dans un second mode, de sorte que, dans le premier mode, la valve (24) est une valve à une voie, qui permet uniquement un écoulement depuis la première sortie (4) vers la seconde sortie (14),
**caractérisé en ce que**, dans le second mode, la valve est une valve à deux voies, de sorte que de l'air peut s'écouler à travers le canal dans les deux directions, et de sorte que la valve est traversée à chaque fois par l'écoulement.

2. Agencement selon la revendication 1,
**caractérisé en ce que** la valve (24) inclut une membrane (26) et est susceptible d'être amenée du premier mode au second mode du fait que la membrane (26) est soit traversée par un élément de perçage (22), soit ouverte par une pression appliquée sur la membrane (26).

3. Système selon l'une des revendications précédentes,
**caractérisé en ce que** ledit au moins un canal (6) comporte une troisième sortie (56) à relier au perçage traversant (42) ménagé dans la tige de prothèse (36) et un élément de terminaison (58), lequel peut être relié à la troisième sortie (56) et, dans une situation reliée, empêche un écoulement entrant via la troisième sortie (56) vers ledit au moins un canal (6).

4. Agencement selon la revendication 3,
**caractérisé en ce que** l'élément de terminaison (58) comprend une valve à une voie (60) qui, dans la situation reliée de l'élément de terminaison (58) avec la troisième sortie (56), autorise uniquement un écoulement sortant via la troisième sortie (56) hors dudit au moins un canal (6).

5. Système avec un agencement selon l'une des revendications précédentes et une pompe avec un boîtier de pompe (18), dans lequel des organes de liaison (12) qui se correspondent mutuellement sont réalisés sur le boîtier de pompe (18) et sur l'agencement, organes au moyen desquels l'agencement est susceptible d'être relié au boîtier de pompe (18).

6. Système selon la revendication 5,
**caractérisé en ce que** les organes de liaison (12) sont réalisés sous la forme d'au moins une gorge (10) et d'une languette (28) correspondant à la gorge (10).

7. Système selon la revendication 5,
**caractérisé en ce que** les organes de liaison (12) incluent une plaque de liaison (50) prévue sur l'agencement.

8. Système selon la revendication 5, 6 ou 7,
**caractérisé en ce qu'**une broche (22) dépassant vers l'extérieur est agencée sur le boîtier de pompe (18), laquelle est réalisée de telle façon qu'elle perce la membrane (26), ou bien que la membrane (26) est ouverte par la pression appliquée sur la membrane (26), quand le boîtier de pompe (18) est relié à l'agencement.

9. Système selon l'une des revendications 5 à 8,
**caractérisé en ce qu'**un élément d'application de force (16) respectif est agencé sur le boîtier de pompe (18) et sur l'agencement, éléments qui provoquent conjointement une force quand le boîtier de pompe (18) est relié à l'agencement, ladite force étant opposée à un détachement du boîtier de pompe (18) depuis l'agencement.

10. Système selon la revendication 9,
**caractérisé en ce qu'**un perçage d'introduction (88) respectif est ménagé sur le boîtier de pompe (18) et sur l'agencement, perçages qui sont alignés l'un avec l'autre dans la situation reliée, de sorte qu'un élément d'enfichage (84) est susceptible d'être passé à travers le perçage d'introduction (88).

11. Système de prothèse comprenant une tige de prothèse, un agencement selon l'une des revendications 1 à 4, et une superstructure de prothèse (20).

12. Système de prothèse comprenant une tige de prothèse, un système selon l'une des revendications 5 à 10, et une superstructure de prothèse (20).
